# EUROPEAN PATENT APPLICATION

(11) **EP 3 402 215 A1**
(43) Date of publication of application: **14.11.2018**
(21) Application number: 18171199.5
(22) Date of filing: 08.05.2018
(51) Int. Cl.: H04R 5/033, H04R 5/04, H04R 29/00, A61B 5/12, H04M 1/24

(54) **SMART HEADPHONE DEVICE PERSONALIZATION SYSTEM AND METHOD FOR USING THE SAME**

(30) Priority: 10.05.2017 TW 106115433
(71) Applicant: Zhao, Ping, Chaoyang District, Beijing (CN); Chang, Ei-Eu, Taipei City (TW)
(72) Inventor: Zhao, Ping, Chaoyang District, Beijing (CN); Chang, Ei-Eu, Taipei City (TW)
(74) Representative: Viering, Jentschura & Partner mbB Patent- und Rechtsanwälte

(57) **Abstract**

Disclosed are a smart headphone device (400) personalization system and a method for using the system. A user downloads a headphone personalization application program unit (200) from the server device (100) for installation in a mobile device (300). The mobile device (300) and the smart headphone device (400) are paired. The user enters corresponding data through the register module (21) of the mobile device (300). The user wears the smart headphone device (400) to allow a hearing inspection module (22) of the mobile device (300) to inspect hearing capabilities of left and right ears of the user and generate an inspection result. The automatic compensation unit (44) of the smart headphone device (400) may automatically carry out compensation according to the inspection result. Then, the manual compensation module (23) of the mobile device (300) further carries out compensation. Finally, the user adjusts the sound effect mode by using the preference setting module (24) of the mobile device (300) to complete personalization of the smart headphone device (400).

## Description

### FIELD OF THE INVENTION

The present invention relates to a smart headphone personalization system and a method for using the system, and in particular to a smart headphone personalization system and a method for using the system that establish and configure a smart headphone device exclusively for the user according to the hearing condition and preference of the user by having a server device, a headphone personalization application program unit, a mobile device, and a smart headphone device operated according to the way of use thereof.

### BACKGROUND OF THE INVENTION

With the continuous progress of the network technology, more and more people use a personal mobile device to listen to music on line or to download audio files to a personal mobile device for subsequent listening. To allow a person to listen to music in a non-disturbed environment at any time and any place, a headphone is becoming an accessory or device that is necessary to a personal mobile device.

To ensure a comfortable condition of listening to music, a person may adjust the broadcasting volume of the headphone to achieve such a purpose. However, some of the known headphone devices only allow a person to make volume adjustment for both left and right ears at the same time, but no concern has been placed on the condition that the hearing capability may be different for the left and right ears, similar to the vision capability being different for the left and right eyes. As a result of such a situation, to prevent damage or loss of hearing, the broadcasting volume must be properly adjusted for different hearing capabilities. However, repeated volume adjustment for the left and right ears separately for each time of use would be very inconvenient and troublesome of the user.

Further, the level of perception of hearing may be different for different persons. Some like high-frequency sound, while the other love heavy base sound. Headphones that are currently known are generally incapable of adjustment that really suits the needs of the users.

In view of the above, it is now a challenging issue to provide a smart headphone device personalization system and a method for using the system that configure a smart headphone device exclusively for a specific user according to a hearing condition and preference of the user.

### SUMMARY OF THE INVENTION

The primary objective of the present invention is to provide a smart headphone device personalization system and a method for using the system, in which a server device, a headphone personalization application program unit, a mobile device, and a smart headphone device are operated according to the way of use thereof to establish and configure a smart headphone device exclusively for the user according to the hearing condition and preference of the user. When the user wears the personalized smart headphone device to listen to music, since issues caused by hearing difference between the two ears of the user have been overcome, it is not necessary to frequently adjust the sound volume and it is also possible to avoid any consequence of deafness caused by listing to music with improper sound volume applying to the ears, and the frequency band of music listening can be adjusted according to the user's preference to allow the user to listen to music in a comfortable and easy condition.

Another objective of the present invention is to provide a smart headphone device personalization system and a method for using the system, in which sampling, analysis, and processing are conducted on external environmental noise and the volume of the headphone unit is adjusted according to the result of analysis to provide a function of easy listening, or a signal that suppresses the external environmental noise developed according to the result of analysis is transmitted the headphone unit to provide a function of active noise cancellation, both allowing the user to clearly listen to music at any time and any place.

A further objective of the present invention is to provide a smart headphone device personalization system and a method for using the system, in which to keep the smart headphone device in an optimum condition for listening, a periodic inspection function is involved for inspection of the variation of hearing of the user and for adjusting the setting of the smart headphone device according to the variation.

Thus, to achieve the above objectives, the present invention provides a smart headphone device personalization system, which comprises: a server device, the server device comprising therein a headphone personalization application program unit, the headphone personalization application program unit comprising a register module, a hearing inspection module, a manual compensation module, and a preference setting module, wherein the register module allows a user to enter corresponding data for registration; the hearing inspection module allows the user to carry out hearing inspection and generates an inspection result; the manual compensation module allows the user to carry out manual compensation according to the inspection result; and the preference setting module allows the user to adjust a sound effect mode according to personal preference, so that when the setting is done, a personalized setting that is fit to the hearing of the user is provided; a mobile device, which is connected, through a first transmission unit, to a network to downward, from the server device, the headphone personalization application program unit for installation in the mobile device; and a smart headphone device, which comprises: a central processing unit; a second transmission unit, which is electrically connected with the central processing unit in order to receive, through pairing between the second transmission unit and the first transmission unit, an instruction transmitted from the mobile device for transmission to the central processing unit, such that the central processing unit converts the instruction into a corresponding signal; a headphone unit, which is electrically connected with the central processing unit so that the headphone unit gives off the corresponding signal to be listened to by the user; an automatic compensation unit, which is electrically connected with the central processing unit and carries out automatic compensation according to the inspection result generated by the hearing inspection module; a storage unit, which is electrically connected with the central processing unit for storing the personalized setting in the storage unit; and a power supply unit, which is electrically connected with the central processing unit to supply electrical power necessary for the smart headphone device.

In the above embodiment, the smart headphone device further comprises: at least a sampling unit, which is adapted to receive an external environmental noise; and a sampling signal processing unit, which is electrically connected with the sampling unit and the central processing unit for analyzing variation of the external environmental noise to generate an analysis signal and transmits the analysis signal to the central processing unit, so that the central processing unit adjusts a broadcasting volume of the headphone unit according to the analysis signal.

In the above embodiment, the smart headphone device further comprises a digital signal processing unit, which is electrically connected with the central processing unit, wherein the digital signal processing unit is operable to adjust, according to the analysis signal, the broadcasting volume and frequency of the headphone unit.

In the above embodiment, the smart headphone device further comprises an amplifier unit, wherein the amplifier unit is electrically connected with the central processing unit and the headphone unit to amplify an audio signal issued from the headphone unit.

In the above embodiment, the smart headphone device further comprises a satellite positioning unit, the satellite positioning unit being electrically connected with the central processing unit, the satellite positioning unit being operable to acquire a site where the smart headphone device is currently located for subsequent transmission to the central processing unit.

In the above embodiment, the register module comprises at least one data field for data entry by a user.

In the above embodiment, the data field comprises user name, password, re-entry of password, gender, age, occupation and smart headphone device serial number.

In the above embodiment, the headphone personalization application program unit further comprises a function selection module, which allows a user to select among functions provided by the smart headphone device.

The present invention also provides a method for using a smart headphone device personalization system, which comprises, at least, the following steps: Step 1: a user downloading a headphone personalization application program unit from a server device for installation in a mobile device for pairing the mobile device with a smart headphone device to complete a pairing procedure; Step 2: conducting a registration procedure, wherein the user enters corresponding data through a register module of the mobile device; Step 3: conducting a hearing inspection procedure, wherein the user wears the smart headphone device and a hearing inspection module of the mobile device is put into operation to inspect hearing capabilities of left and right ears of the user and generate an inspection result; Step 4: conducting a hearing compensation procedure, wherein an automatic compensation unit of the smart headphone device is first operated to automatically carry out compensation according to the inspection result and then, the user operates a manual compensation module of the mobile device to carry out compensation; and Step 5: conducting a preference setting procedure, wherein the user operates a preference setting module of the mobile device to adjust a sound effect mode, so that after the setting is done, personalization of the smart headphone device is completed.

In the above embodiment, the register module comprises at least one data field for data entry by a user.

In the above embodiment, the data field the data field comprises user name, password, re-entry of password, gender, age, occupation and smart headphone device serial number.

In the above embodiment, the hearing inspection procedure further comprises a standby state and an inspection state.

In the above embodiment, in the standby state, a level of volume of the smart headphone device is turned down to a minimum value and the sampling unit of the smart headphone device is adjusted to a predetermined position in order to detect an environmental noise, wherein when the environmental noise is detected to be of a relative low level, switching is made to the inspection state, or otherwise, when the environmental noise is detected to be of a relatively high level, no switching is made to the inspection state.

In the above embodiment, in the inspection state, the user hears through the smart headphone device audio signals issued from the mobile device, wherein the user is allowed to hear the audio signal each time with just one ear, wherein the user, upon hearing the audio signal, actuates a confirmation button on the mobile device to issue a confirmation message, and the mobile device subsequently issue a next one of audio signal having a different frequency or volume, wherein after multiple times of inspection, an inspection result is displayed on the mobile device and the inspection result is stored in the storage unit and uploaded through the mobile device to the server device for storage.

In the above embodiment, after the inspection of both ears of the user is done and the inspection result is generated, then the hearing compensation procedure is conducted, wherein the hearing compensation procedure further comprises auditory curve compensation, auditory threshold sensitivity compensation, balance compensation, and position recognition compensation, wherein the auditory curve compensation and the auditory threshold sensitivity compensation are automatic compensation conducted with the automatic compensation unit and the balance compensation and the position recognition compensation are compensation conducted with the manual compensation module by the user.

In the above embodiment, the sound effect mode comprises flat mode, news mode, classic mode, fashion mode, rock-and-roll mode, and heavy bass mode to be selected by the user.

In the above embodiment, further included is Step 6: conducting a function selection procedure to allow the user to make selection, through a function selection module, among functions provided by the smart headphone device.

In the above embodiment, the functions comprise an active noise cancellation function and an easy listening function.

### BRIEF DESCRIPTION OF THE DRAWINGS

The present invention will be apparent to those skilled in the art by reading the following description of preferred embodiments thereof with reference to the drawings, in which:
FIG. 1 is a schematic view illustrating architecture of a smart headphone device personalization system according to the present invention;
FIG. 2 is a schematic view a function module of a headphone personalization application program unit according to the present invention;
FIG. 3 is a schematic view illustrating a registration module of FIG. 2;
FIGS. 4A-4D are schematic views illustrating a hearing inspection module of FIG. 2;
FIGS. 5A-5C are schematic views illustrating a manual compensation module of FIG. 2;
FIG. 6 is a schematic view illustrating a preference setting module of FIG. 2;
FIG. 7 is a schematic view illustrating another architecture of the smart headphone device personalization system according to the present invention;
FIG. 8 is flow chart illustrating a method for using the smart headphone device personalization system according to the present invention; and
FIG. 9 is a schematic view illustrating operation of a function selection module with the method for using the smart headphone device personalization system according to the present invention.

### DETAILED DESCRIPTION OF THE PREFERRED EMBODIMENTS

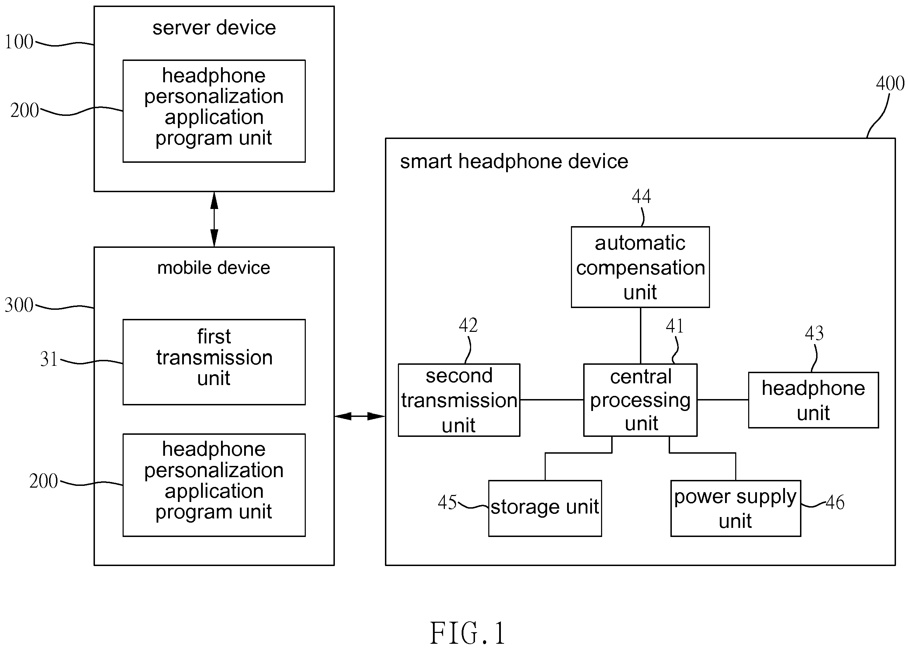

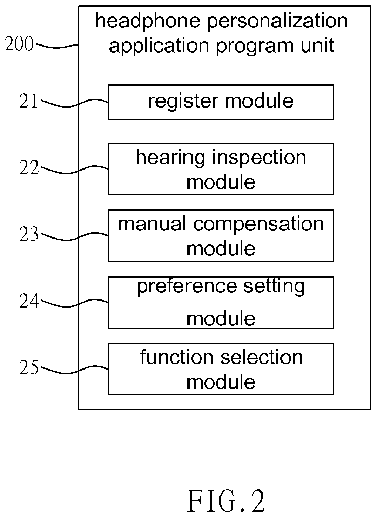

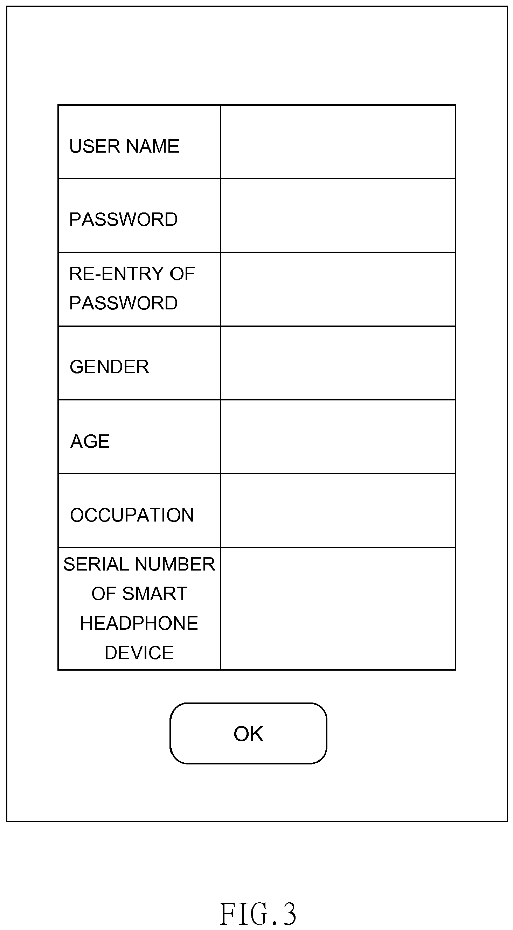

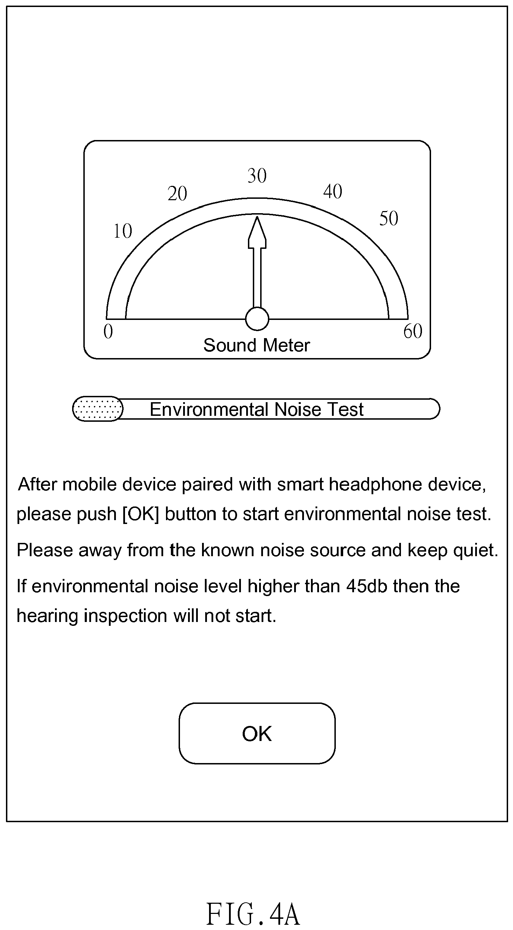

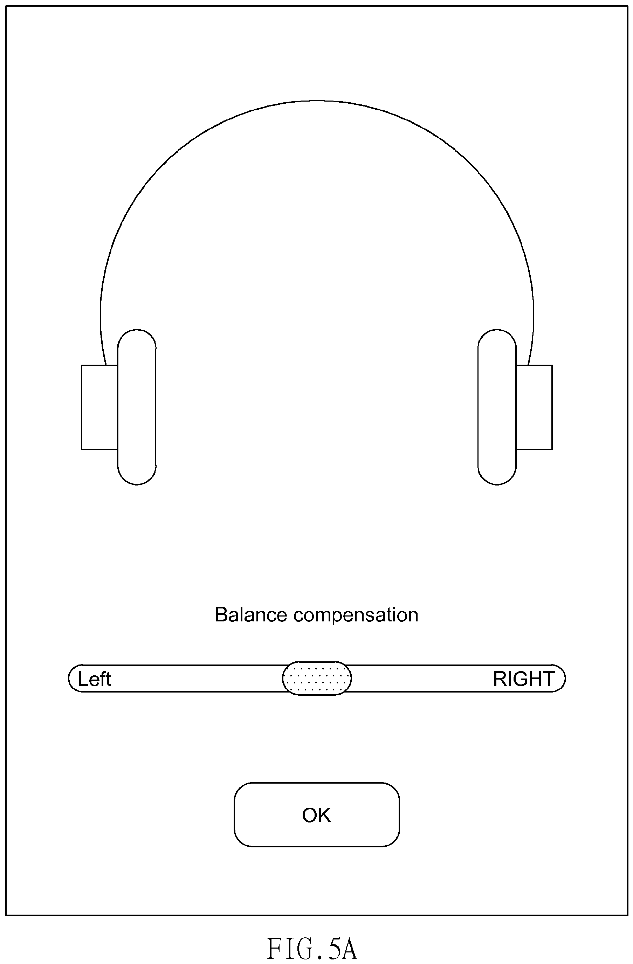

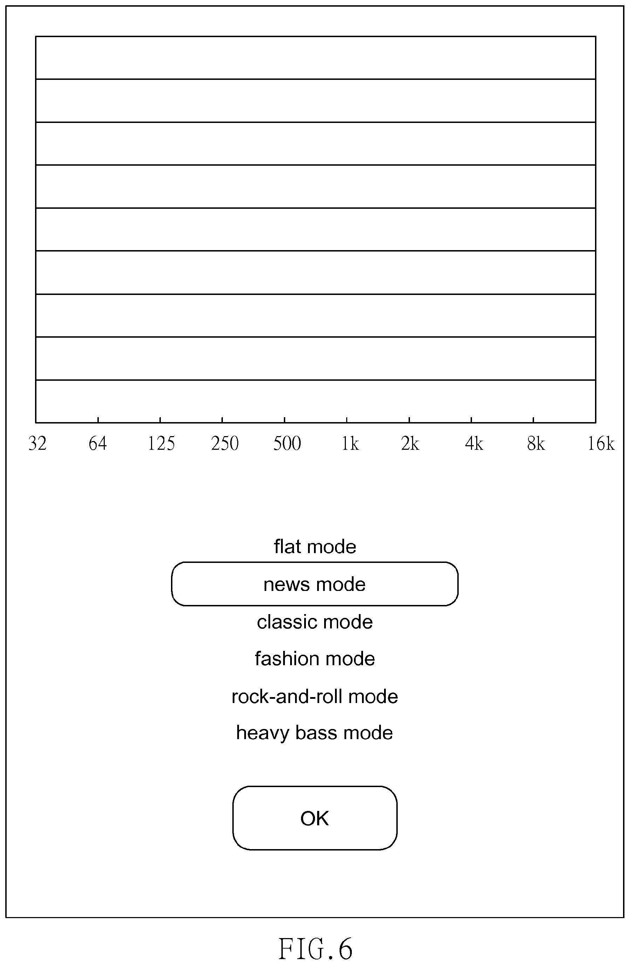

With reference to FIGS. 1-6, a smart headphone device personalization system according to the present invention comprises:
a server device 100, the server device 100 comprising a headphone personalization application program unit 200 stored therein, the headphone personalization application program unit 200 comprising a register module 21, a hearing inspection module 22, a manual compensation module 23, and a preference setting module 24, wherein the register module 21 allows a user to enter corresponding data for registration (wherein, as shown in FIG. 3, the register module 21 comprises at least one data field to data entry by the user, wherein the data field comprises user name, password, re-entry of password, gender, age, occupation, and serial number of a smart headphone device 400); the hearing inspection module 22 allows the user to conduct hearing inspection and generates an inspection result (as shown in FIGS. 4A-4D); the manual compensation module 23 allows the user to do manual compensation for the inspection result (as shown in FIGS. 5A-5C); and the preference setting module 24 allows the user to adjust a sound effect mode according to personal preference (as shown in FIG. 6), whereby after the process is completed, a personalized setting that is specifically fit for the user's hearing and in addition, the headphone personalization application program unit further comprises a function selection module 25, which allows the user make selection among functions provided by the smart headphone device 400;
a mobile device 300 (which can be a mobile device that is for example a smart phone, a tablet computer, and the likes), which is connected through a first transmission unit 31 to a network in order to download the headphone personalization application program unit 200 from the server device 100 for installation in the mobile device 300; and
a smart headphone device 400, which comprises a central processing unit 41; a second transmission unit 42, which is electrically connected with the central processing unit 41 in order to receive, through pairing between the second transmission unit 42 and the first transmission unit 31, an instruction transmitted from the mobile device 300 for transmission to the central processing unit 41, such that the central processing unit 41 converts the instruction into a corresponding signal; a headphone unit 43, which is electrically connected with the central processing unit 41 so that the headphone unit 43 gives off the corresponding signal to be listened to by the user; an automatic compensation unit 44, which is electrically connected with the central processing unit 41 and carries out automatic compensation according to the inspection result generated by the hearing inspection module; a storage unit 45, which is electrically connected with the central processing unit 41 for storing the personalized setting in the storage unit; and a power supply unit 46, which is electrically connected wit the central processing unit 41 to supply electrical power necessary for the smart headphone device 400, wherein the smart headphone device 400 can be a circumaural Bluetooth headphone.

Thus, the user is allowed to use the smart headphone device personalization system to establish or configure a smart headphone device exclusively for himself or herself. When the user wears the personalized smart headphone device in connection with a sound playing device, such as a mobile device, a tabletop computer, a notebook computer, or a portable playing device, to listen to music or radio broadcasting, since any issue caused by hearing difference between the two ears of the user has been overcome, it is not necessary to constantly adjust the sound volume and it is also possible to avoid any consequence of deafness caused by listing to music with improper sound volume applying to the ears, and the frequency band of music listening can be adjusted according to the user' s preference to allow the user to listen to music in a comfortable and easy condition.

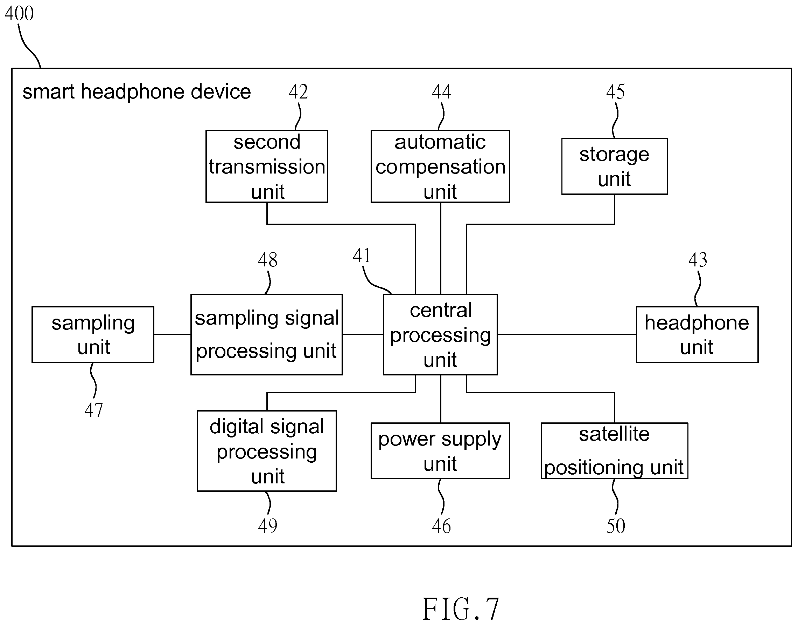

Next, referring to FIG. 7, the smart headphone device 400 further comprises at least a sampling unit 47, which is a device for receiving and collecting sound and is provided for receiving external environmental noise; and a sampling signal processing unit 48, which is electrically connected with the sampling unit 47 and the central processing unit 41 for analyzing variation of the external environmental noise to generate an analysis signal and transmits the analysis signal to the central processing unit 41, so that the central processing unit 41 may adjust the broadcasting volume of the headphone unit 43 according to the analysis signal in order to prevent the user from comfortably and easily listening to music due to perception of different levels of sound, such as excessively loud or excessively low, in different environments.

In addition, the smart headphone device 400 further comprises a digital signal processing unit 49, which is electrically connected with the central processing unit 41, wherein the digital signal processing unit 49 may provide, according to the analysis signal, a reversed signal of a proper ratio or percentage to the headphone unit 43, so that after sampling of the external environmental noise carried out by the sampling unit 47, transmission is made to the sampling signal processing unit 48 for analysis and generation of the analysis signal, and finally, the digital signal processing unit 49 supplies the reversed signal to the headphone unit 43 to realize noise cancellation for the headphone unit to thereby prevent the user, when listening to music, from being affected by the external environmental noise for clearly listening to the music.

In addition, the smart headphone device 400 further comprises a satellite positioning unit 50, wherein the satellite positioning unit 50 is electrically connected with the central processing unit 41, so that the satellite positioning unit 50 may acquire the site where the smart headphone device 400 is currently located for subsequent transmission to the central processing unit 41, whereby when the user wears and uses the smart headphone device 400, the site of the user can be clearly identified.

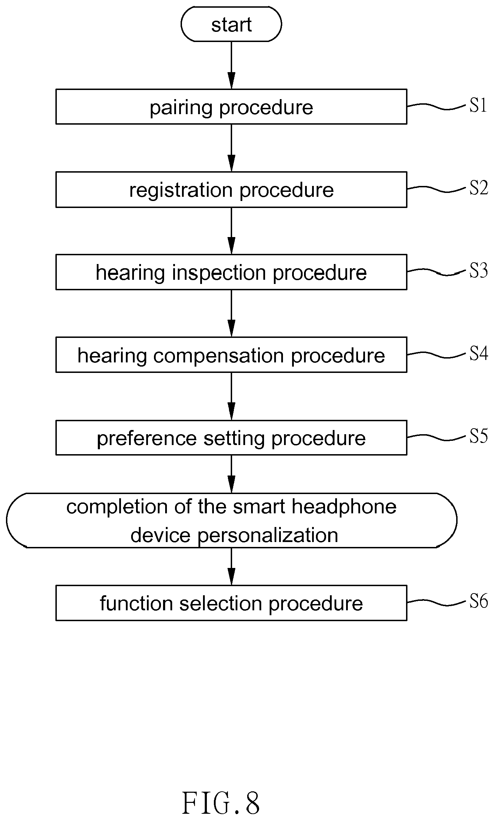

Also referring to FIG. 8, a method for using the smart headphone device personalization system according to the present invention is illustrated, comprising at least the following steps: Step one (S1), a user downloading a headphone personalization application program unit from a server device for installation in a mobile device (which can be a mobile device that is for example a smart phone, a tablet computer, and the likes) and for pairing the mobile device with a smart headphone device to complete a pairing procedure; Step two (S2), conducting a registration procedure, wherein the user enters corresponding data through the register module of the mobile device; Step three (S3), conducting a hearing inspection procedure, wherein the user wears the smart headphone device and a hearing inspection module of the mobile device is put into operation to inspect the hearing capabilities of left and right ears of the user and generate an inspection result; Step four (S4), conducting a hearing compensation procedure, wherein an automatic compensation unit of the smart headphone device is first operated to automatically carry out compensation according to the inspection result and then, the user operates a manual compensation module of the mobile device to carry out compensation; and Step five (S5), conducting a preference setting procedure, wherein the user operates a preference setting module of the mobile device to adjust a sound effect mode, so that after the setting is done, personalization of the smart headphone device is completed.

A detailed description is provided below:
Firstly, after the mobile device and the smart headphone device complete the pairing procedure, a registration procedure is then carried out, wherein the register module comprises at least one data field to allow the user to make entry of data thereto (as shown in FIG. 3), and the data field comprises user name, password, re-entry of password, gender, age, occupation, and serial number of smart headphone device. The user must complete entry of each piece of data into the data field to complete registration.

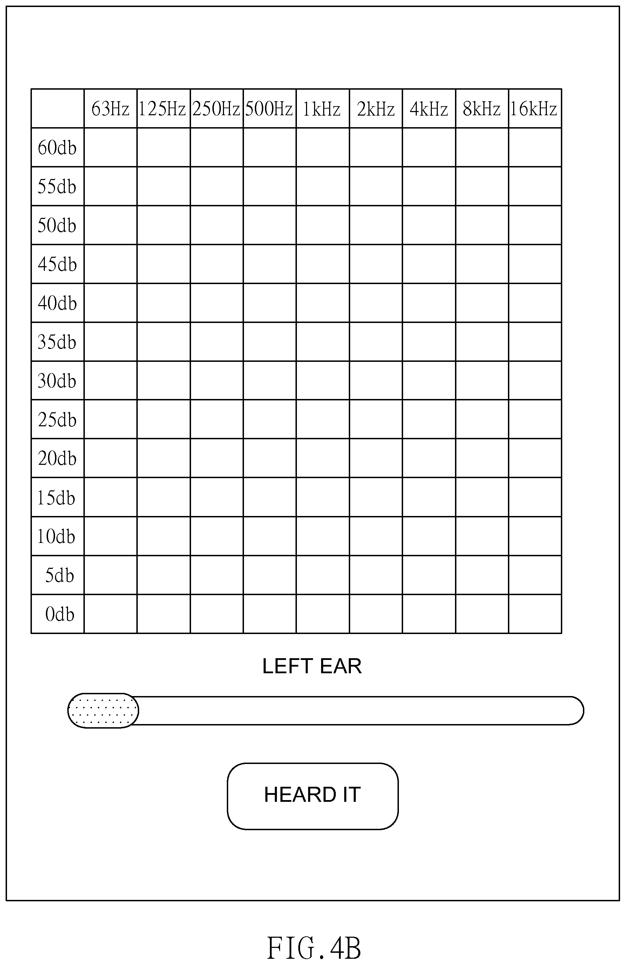

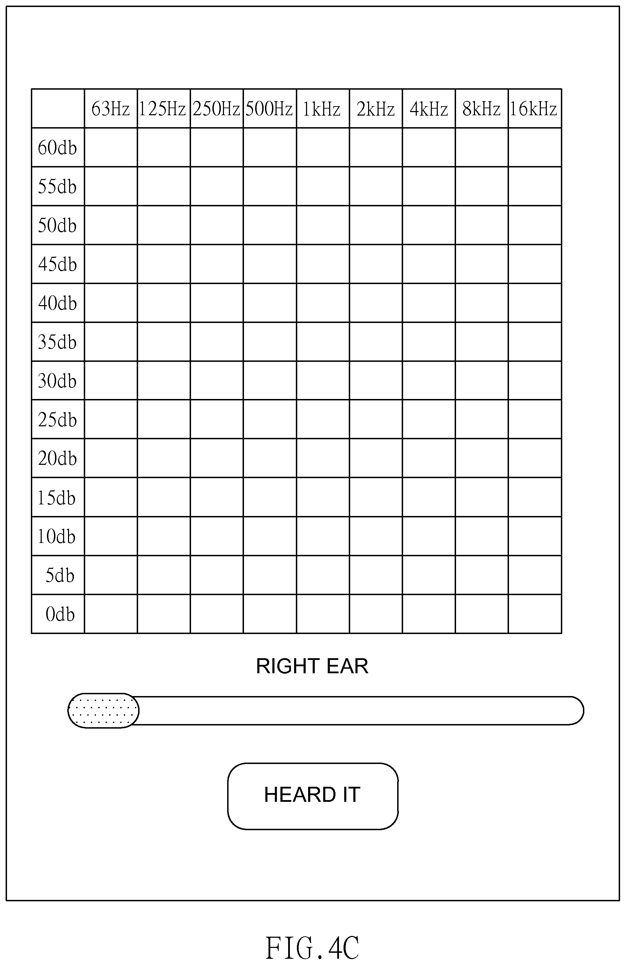

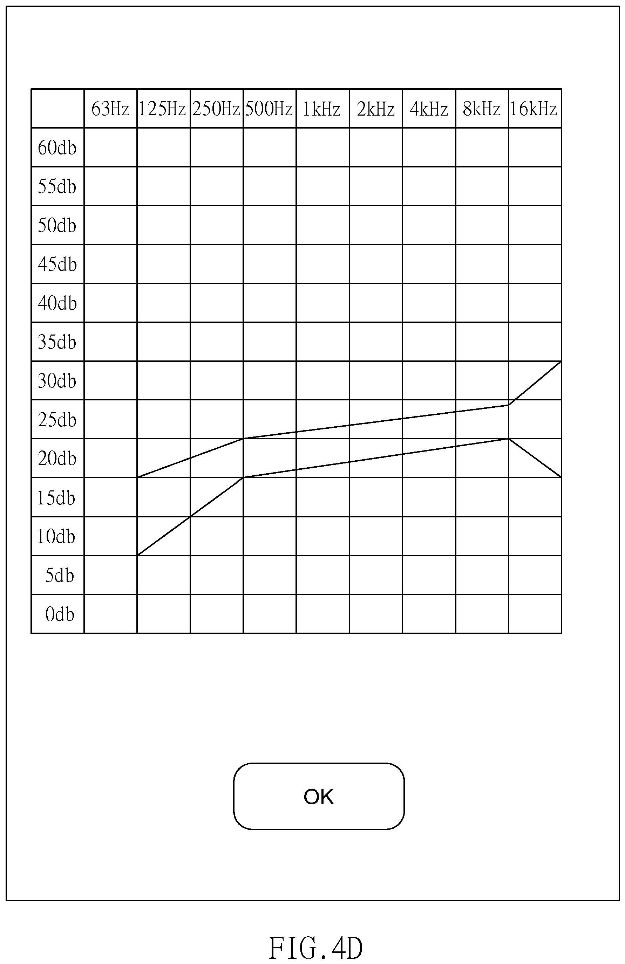

After completion of the registration procedure, hearing inspection must be done on the user so that a hearing inspection procedure is carried out, wherein the hearing inspection procedure further comprises a standby state and an inspection state, wherein in the standby state (as shown in FIG. 4A), a level of volume of the smart headphone device is turned down to the minimum and the sampling unit of the smart headphone device is adjusted to a predetermined position in order to detect the environmental noise, wherein when the environmental noise is detected to be of a relative low level, switching is made to the inspection state, or otherwise, when the environmental noise is detected to be of a relatively high level, no switching is made to the inspection state. In switching to the inspection state (as shown in FIGS. 4B and 4C), the user will hear, through the smart headphone device, audio signals issued from the mobile device, wherein the frequency or volume is different among the audio signals and the user is allowed to hear the audio signal each time with just one ear. The user, upon hearing the audio signal, actuates a confirmation button on the mobile device (such as the "confirmation" button shown in FIGS. 4B and 4C) to issue a confirmation message, and the mobile device will then issue an audio signal having a different frequency or volume from that audio signal. After several times of inspection so conducted, the inspection result will be shown on the mobile device (such as the hearing curves of left and right ears shown in FIG. 4D), and the inspection result is stored in the storage unit and uploaded through the mobile device to the server device for storage therein.

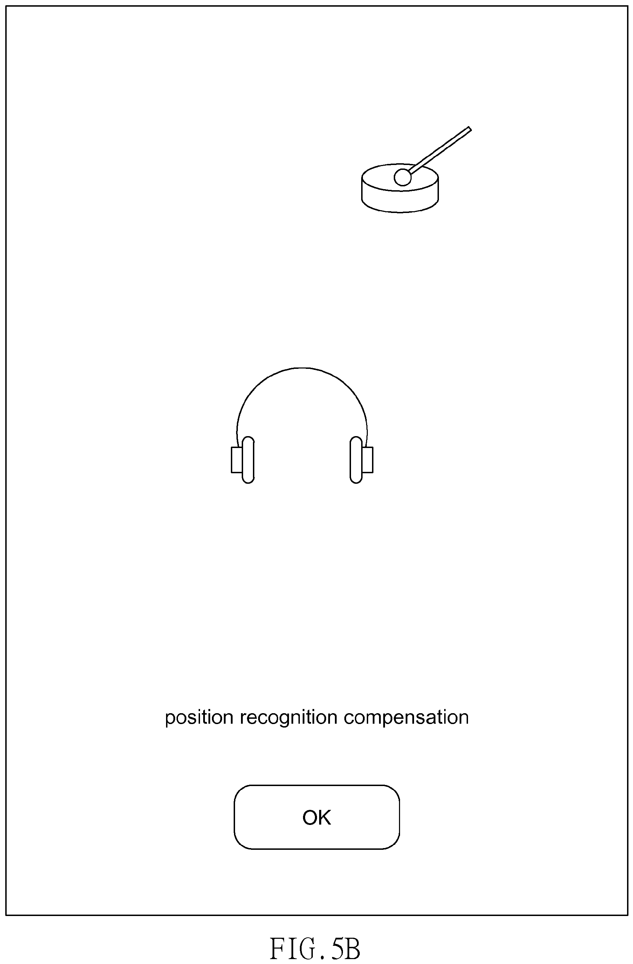

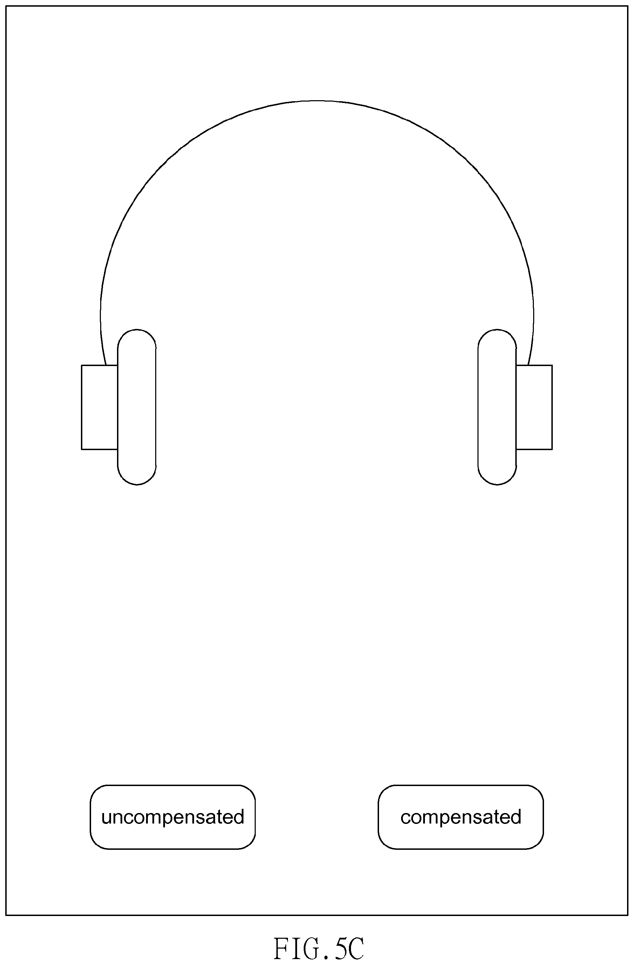

Thus, after both ears of the user have been inspected and the inspection result have been generated, the hearing compensation procedure is then conducted, wherein firstly, the automatic compensation unit of the smart headphone device carries out automatic compensation according to the inspection result and is pre-set with a listening volume and the user carries out further compensation with the manual compensation module of the mobile device, wherein the hearing compensation procedure further comprises auditory curve compensation, auditory threshold sensitivity compensation, balance compensation (as shown in FIG. 5A), and position recognition compensation (as shown in FIG. 5B). The auditory curve compensation and the auditory threshold sensitivity compensation are automatic compensation conducted with the automatic compensation unit, for example for minute adjustment for trend and the headphone personalization application program unit automatically determining which ear is taken as reference, wherein if the inspection result indicates treble of the right ear and bass of the left ear are normal, then compensation can be made just for bass of the right ear and treble of the left ear; the balance compensation and the position recognition compensation are compensation conducted with the manual compensation module by the user, allowing the user to do minute adjustment to such an extent that sound is perceived as emission from a center of the head, and then one segment of music that has been compensated and one segment of music that has not been compensated will be played to the user so that the user may determine if to activate the compensation function (as shown in FIG. 5C).

The preference setting procedure is carried out finally. The user, based on his or her preference, operates the preference setting module of the mobile device to adjust a sound effect mode (as shown in FIG. 6), wherein the sound effect mode comprises news mode, classic mode, fashion mode, rock-and-roll mode, and heavy bass mode to be selected by the user. The personalization of the smart headphone device is completed after the setting is done. Thus, the method for using can be applied to make the smart headphone device correct the hearing of the user. When the user has the personalization-completed smart headphone device connected with a sound playing device, such as a mobile device, a tabletop computer, a notebook computer, or a portable playing device, to listen to music or radio broadcasting, since any issue caused by hearing difference between the two ears of the user has been overcome, it is not necessary to frequently adjust the sound volume and it is also possible to avoid any consequence of deafness caused by listing to music with improper sound volume applying to the ears, and the frequency band of music listening can be adjusted according to the user' s preference to allow the user to listen to music in a comfortable and easy condition and to achieve a good effect of music listening.

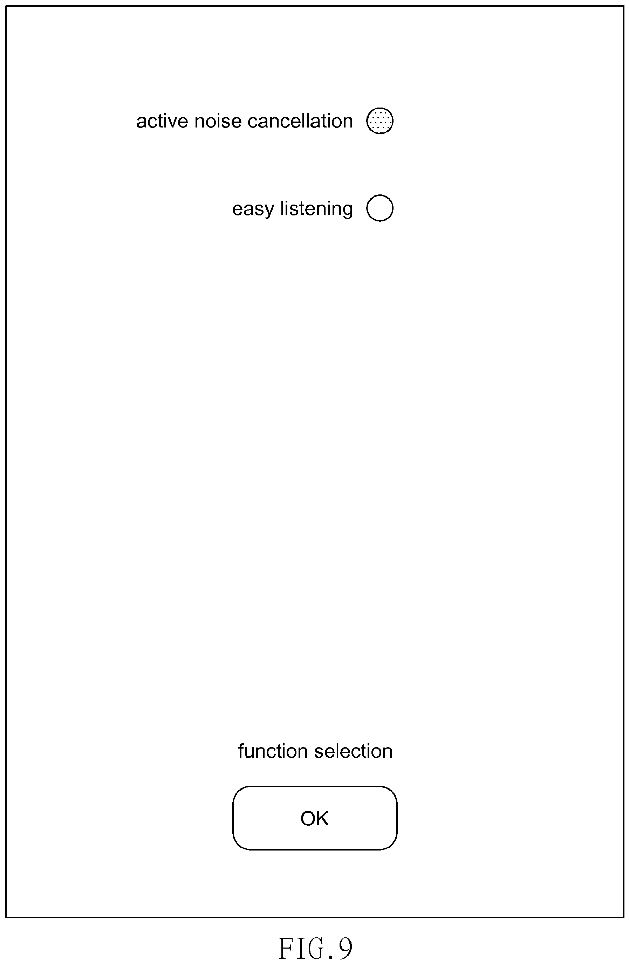

In addition, the method for using the smart headphone device personalization system according to the present invention may further comprises Step six (S6), conducting a function selection procedure to allow the user to make selection, through a function selection module, among functions provided by the smart headphone device, as shown in FIG. 9, wherein the functions comprise an active noise cancellation function and an easy listening function between which switching can be made by the user. The active noise cancellation function is such that the sampling unit makes sampling of the external environmental noise for transmission to the sampling signal processing unit for analysis and generation of an analysis signal, and finally, the digital signal processing unit supplies a reversed signal to he headphone unit to prevent the user from being affected by the external environmental noise in listening to music so that music can be clearly heard. The easy listening function is such that the sampling unit makes sampling of the external environmental noise for transmission to the sampling signal processing unit for analysis and generation of an analysis signal, and then, the central processing unit makes adjustment, according to the analysis signal, on the broadcasting volume of the headphone unit so that when variation occurs on the environmental noise (such as the noise being increased or decreased) during the user listening to music, the smart headphone device may timely adjust the volume of broadcasting (volume being increased or decreased) made by the headphone unit in order to allow the user to listen to music easily and clearly.

Further, when the user has continuously listened to music for a first preset time (such as 60 minutes), the headphone personalization application program unit stored in the mobile device automatically issues a reminder message. For example, a message of "rest for 10 minutes is recommended" is displayed on a screen of the mobile device, and at the same time, the headphone unit of the smart headphone device gives off a special sound of reminder. However, when listening has been continued to exceed a second preset time (such as 120 minutes), the headphone personalization application program unit stored in the mobile device also automatically issues a reminder message. Similarly, a message of "rest for 10 minutes is recommended" is displayed on the screen of the mobile device and the headphone unit of the smart headphone device gives off a special sound of reminder. If listening has been further made for a third preset time (such as 5 minutes), the smart headphone device automatically shuts down and thus providing a reminder function of health-based listening.

Finally, when the smart headphone device has been successively used for more than a predetermined number of days (such as 90 days), the screen of the mobile device will display, upon powering on, a reminder, such as "please have your hearing level inspected" , and thus, the user has to go over the operations of inspection and adjustment through Step two (S2) to Step five(S5). The results of inspection and adjustment for each time will be stored in the storage unit of the smart and the server device in order to provide periodic inspection of variation of hearing of the user and adjustment of the setting of the smart headphone device according thereto for keeping the smart headphone device in an optimum condition of listening.

In summary, the present invention provides a smart headphone device personalization system and a method for using the system, in which a server device, a headphone personalization application program unit, a mobile device, and a smart headphone device are operated according to the way of use thereof (pairing, registration, hearing inspection, hearing compensation, and preference setting) to establish and configure a smart headphone device exclusively for the user according to the hearing condition and preference of the user. When the user wears the personalized smart headphone device to listen to music, since issues caused by hearing difference between the two ears of the user have been overcome, it is not necessary to frequently adjust the sound volume and it is also possible to avoid any consequence of deafness caused by listing to music with improper sound volume applying to the ears, and the frequency band of music listening can be adjusted according to the user's preference to allow the user to listen to music in a comfortable and easy condition. Further, the present invention further provides a smart headphone device personalization system and a method for using the system, in which sampling, analysis, and processing are conducted on external environmental noise and the volume of the headphone unit is adjusted according to the result of analysis to provide a function of easy listening, or a signal that suppresses the external environmental noise developed according to the result of analysis is transmitted the headphone unit to provide a function of active noise cancellation, both allowing the user to clearly listen to music at any time and any place. Finally, to keep the smart headphone device in an optimum condition for listening, a periodic inspection function is involved for inspection of the variation of hearing of the user and for adjusting the setting of the smart headphone device according to the variation.

Although the present invention has been described with reference to the preferred embodiments thereof, it is apparent to those skilled in the art that a variety of modifications and changes may be made without departing from the scope of the present invention which is intended to be defined by the appended claims.

## Claims

1. A smart headphone device (400) personalization system, comprising:
a server device (100), the server device (100) comprising therein a headphone personalization application program unit (200), the headphone personalization application program unit comprising a register module (21), a hearing inspection module (22), a manual compensation module (23), and a preference setting module (24), wherein:
the register module (21) allows a user to enter corresponding data for registration;
the hearing inspection module (22) allows the user to carry out hearing inspection and generates an inspection result;
the manual compensation module (23) allows the user to carry out manual compensation according to the inspection result; and
the preference setting module (24) allows the user to adjust a sound effect mode according to personal preference, so that when the setting is done, a personalized setting that is fit to the hearing of the user is provided;
a mobile device (300), which is connected, through a first transmission unit (31), to a network to downward, from the server device (100), the headphone personalization application program unit (200) for installation in the mobile device (300); and
a smart headphone device (400), comprising:
a central processing unit (41);
a second transmission unit (42), which is electrically connected with the central processing unit (41) in order to receive, through pairing between the second transmission unit (42) and the first transmission unit (31), an instruction transmitted from the mobile device (300) for transmission to the central processing unit (41), such that the central processing unit (41) converts the instruction into a corresponding signal;
a headphone unit (43), which is electrically connected with the central processing unit (41) so that the headphone unit (43) gives off the corresponding signal to be listened to by the user;
an automatic compensation unit (44), which is electrically connected with the central processing unit (41) and carries out automatic compensation according to the inspection result generated by the hearing inspection module (22);
a storage unit (45), which is electrically connected with the central processing unit (41) for storing the personalized setting in the storage unit (45); and
a power supply unit (46), which is electrically connected with the central processing unit (41) to supply electrical power necessary for the smart headphone device (400).

2. The smart headphone device (400) personalization system according to Claim **1**, wherein the smart headphone device (400) further comprises:
at least a sampling unit (47), which is adapted to receive an external environmental noise; and
a sampling signal processing unit (48), which is electrically connected with the sampling unit (47) and the central processing unit (41) for analyzing variation of the external environmental noise to generate an analysis signal and transmits the analysis signal to the central processing unit (41), so that the central processing unit (41) adjusts a broadcasting volume of the headphone unit (43) according to the analysis signal.

3. The smart headphone device (400) personalization system according to Claim **2**, wherein the smart headphone device (400) further comprises a digital signal processing unit (49), which is electrically connected with the central processing unit (41), wherein the digital signal processing unit (49) provides, according to the analysis signal, a reversed signal to the headphone unit (43).

4. The smart headphone device (400) personalization system according to Claim **1**, **2**, or **3**, wherein the smart headphone device (400) further comprises a satellite positioning unit (50), the satellite positioning unit (50) being electrically connected with the central processing unit (41), the satellite positioning unit (50) being operable to acquire a site where the smart headphone device (400) is currently located for subsequent transmission to the central processing unit (41).

5. The smart headphone device (400) personalization system according to Claim **1**, wherein the register module (21) comprises at least one data field for data entry by a user.

6. The smart headphone device (400) personalization system according to Claim **5**, wherein the data field comprises user name, password, re-entry of password, gender, age, occupation and smart headphone device (400) serial number.

7. The smart headphone device (400) personalization system according to Claim **1**, wherein the headphone personalization application program unit (200) further comprises a function selection module (25), which allows a user to select among functions provided by the smart headphone device (400).

8. A method for using a smart headphone device (400) personalization system, comprising at least the following steps:
Step 1 (S1): a user downloading a headphone personalization application program unit (200) from a server device (100) for installation in a mobile device (300) for pairing the mobile device (300) with a smart headphone device (400) to complete a pairing procedure;
Step 2 (S2): conducting a registration procedure, wherein the user enters corresponding data through a register module (21) of the mobile device (300);
Step 3 (S3) : conducting a hearing inspection procedure, wherein the user wears the smart headphone device (400) and a hearing inspection module (22) of the mobile device (300) is put into operation to inspect hearing capabilities of left and right ears of the user and generate an inspection result;
Step 4 (S4): conducting a hearing compensation procedure, wherein an automatic compensation unit (44) of the smart headphone device (400) is first operated to automatically carry out compensation according to the inspection result and then, the user operates a manual compensation module (23) of the mobile device (300) to carry out compensation; and
Step 5 (S5) : conducting a preference setting procedure, wherein the user operates a preference setting module (24) of the mobile device (300) to adjust a sound effect mode, so that after the setting is done, personalization of the smart headphone device (400) is completed.

9. The method for using the smart headphone device (400) personalization system according to Claim **8**, wherein the register module (21) comprises at least one data field for data entry by a user.

10. The method for using the smart headphone device (400) personalization system according to Claim **9**, wherein the data field comprises user name, password, re-entry of password, gender, age, occupation and smart headphone device serial number.

11. The method for using the smart headphone device (400) personalization system according to Claim **8**, wherein the hearing inspection procedure further comprises a standby state and an inspection state.

12. The method for using the smart headphone device (400) personalization system according to Claim **11**, wherein in the standby state, a level of volume of the smart headphone device (400) is turned down to a minimum value and the sampling unit (47) of the smart headphone device (400) is adjusted to a predetermined position in order to detect an environmental noise, wherein when the environmental noise is detected to be of a relative low level, switching is made to the inspection state, or otherwise, when the environmental noise is detected to be of a relatively high level, no switching is made to the inspection state.

13. **The** method for using the smart headphone device (400) personalization system according to Claim **12**, wherein in the inspection state, the user hears through the smart headphone device (400) audio signals issued from the mobile device (300), wherein the user is allowed to hear the audio signal each time with just one ear, wherein the user, upon hearing the audio signal, actuates a confirmation button on the mobile device (300) to issue a confirmation message, and the mobile device (300) subsequently issue a next one of audio signal having a different frequency or volume, wherein after multiple times of inspection, an inspection result is displayed on the mobile device (300) and the inspection result is stored in the storage unit (45) and uploaded through the mobile device (300) to the server device (100) for storage.

14. **The** method for using the smart headphone device (400) personalization system according to Claim **13**, wherein after the inspection of both ears of the user is done and the inspection result is generated, then the hearing compensation procedure is conducted, wherein the hearing compensation procedure further comprises auditory curve compensation, auditory threshold sensitivity compensation, balance compensation, and position recognition compensation, wherein the auditory curve compensation and the auditory threshold sensitivity compensation are automatic compensation conducted with the automatic compensation unit (44) and the balance compensation and the position recognition compensation are compensation conducted with the manual compensation module (23) by the user.

15. The method for using the smart headphone device (400) personalization system according to Claim **8**, wherein the sound effect mode comprises news mode, classic mode, fashion mode, rock-and-roll mode, and heavy bass mode to be selected by the user.

16. The method for using the smart headphone device (400) personalization system according to Claim **8** further comprises Step 6 (S6): conducting a function selection procedure to allow the user to make selection, through a function selection module (25), among functions provided by the smart headphone device (400).

17. The method for using the smart headphone device (400) personalization system according to Claim **16**, wherein the functions comprise an active noise cancellation function and an easy listening function.
